# EUROPEAN PATENT APPLICATION

(11) **EP 4 316 609 A1**
(43) Date of publication of application: **07.02.2024**
(21) Application number: 22779823.8
(22) Date of filing: 07.03.2022
(51) Int. Cl.: A63B 21/002, A63B 23/04, A61N 1/36

(54) **MUSCLE ELECTRICAL STIMULATION PROGRAM AND ELECTRICAL STIMULATION DEVICE**

(30) Priority: 29.03.2021 JP 2021055149
(71) Applicant: MTG Co., Ltd., Nagoya-shi, Aichi 453-0041 (JP)
(72) Inventor: MATSUMOTO Shigeru, Nagoya-shi Aichi 453-0041 (JP)
(74) Representative: Müller-Boré & Partner Patentanwälte PartG mbB
(86) International application number: PCT/JP2022/009595
(87) International publication number: WO 2022/209599

(57) **Abstract**

A muscle electrostimulation program for giving electrostimulation to a muscle, including: a module implemented by a computer of an electrostimulation device provided with a signal application unit that applies a voltage signal to an electrode group, the module being: a module that repeatedly applies a plurality of pulse waveform groups to the electrode group as the voltage signal by controlling the signal application unit, wherein the plurality of pulse waveform groups include: a first pulse waveform group applied intermittently every first cycle corresponding to a frequency of 15 Hz to 25 Hz; and a second pulse waveform group applied outside an application period of the first pulse waveform group in the first cycle.

## Description

### [TECHNICAL FIELD]

The present disclosure relates to an electrostimulation device for giving electrostimulation to a user.

### [BACKGROUND ART]

Patent literature 1 discloses an electrostimulation device including a group of electrodes for giving electrostimulation to both legs of a user. Giving electrostimulation to both legs by using this electrostimulation device allows exercise that involves muscle contraction and relaxation to be encouraged.

[Patent Literature 1] Japanese Patent Application Publication No. 2020-10961

### [SUMMARY OF INVENTION]

### [TECHNICAL PROBLEM]

Recently, there are concerns about chronic lack of exercise among the elderly, etc. In order to solve the lack of exercise, it is desirable to practice ingenuity for encouraging the continuous use of electrostimulation devices that can be used indoors. In order to practice such ingenuity, the inventors of the present application have come to recognize that it is important to diversify physical sensations that can be given to users.

One of the purposes of the present disclosure is to provide a technology that enables diversification of physical sensations that can be given to users using electrostimulation devices.

### [SOLUTION TO PROBLEM]

A program according to the present disclosure is a muscle electrostimulation program for giving electrostimulation to a muscle, including: a module implemented by a computer of an electrostimulation device provided with a signal application unit that applies a voltage signal to an electrode group, the module being: a module that repeatedly applies a plurality of pulse waveform groups to the electrode group as the voltage signal by controlling the signal application unit, wherein the plurality of pulse waveform groups include: a first pulse waveform group applied intermittently every first cycle corresponding to a frequency of 15 Hz to 25 Hz; and a second pulse waveform group applied outside an application period of the first pulse waveform group in the first cycle.

The electrostimulation device according to the present disclosure includes: an electrode group that gives electrostimulation to a user; a signal application unit that applies a voltage signal to the electrode group; and a control unit that executes the muscle electrostimulation program.

### [ADVANTAGEOUS EFFECTS OF INVENTION]

According to the present disclosure, physical sensations that can be given to users using electrostimulation devices can be diversified.

### [BRIEF DESCRIPTION OF DRAWINGS]

FIG. 1 is a configuration diagram of an electrostimulation system according to an embodiment;
FIG. 2 is a right side view showing an electrostimulation device according to the embodiment;
FIG. 3A is an explanatory diagram showing a state in which an EMS waveform group for a left leg is applied to an electrode group; FIG. 3B is an explanatory diagram showing a state in which an EMS waveform group for a right leg is applied to the electrode group;
FIG. 4 is a block diagram showing functions of the electrostimulation device according to the embodiment;
FIG. 5 is a waveform diagram showing the EMS waveform group for the left leg and the EMS waveform group for the right leg;
FIG. 6 is a waveform diagram for first and second pulse waveform groups;
FIG. 7 is a waveform diagram for a pulse waveform group;
Fig. 8 is a waveform diagram for an EMS waveform group applied in a skip mode;
FIG. 9 is a graph showing the results of muscle fatigue measurement taken before and after the use of a walking mode;
FIG. 10 is a graph showing the results of gastrocnemius muscle strength measurement taken before and after a test period for the walking mode;
FIG. 11 shows graphs showing the results of walking speed measurement taken before and after the test period for the walking mode;
FIG. 12 is a graph showing the results of a two-step test performed before and after the test period for the walking mode; and
FIG. 13 is a block diagram showing the functions of a remote controller according to the embodiment.

### [DESCRIPTION OF EMBODIMENTS]

Embodiments will be explained in the following. Like numerals represent like constituting elements, and duplicative explanations will be omitted. For the sake of ease of explanation, constituting elements are appropriately omitted, enlarged, or reduced in the figures. The figures shall be viewed in accordance with the orientation of reference numerals. Vertical and horizontal axes in waveform diagrams are enlarged or reduced as appropriate for ease of understanding. Waveforms in waveform diagrams are simplified as appropriate for ease of understanding.

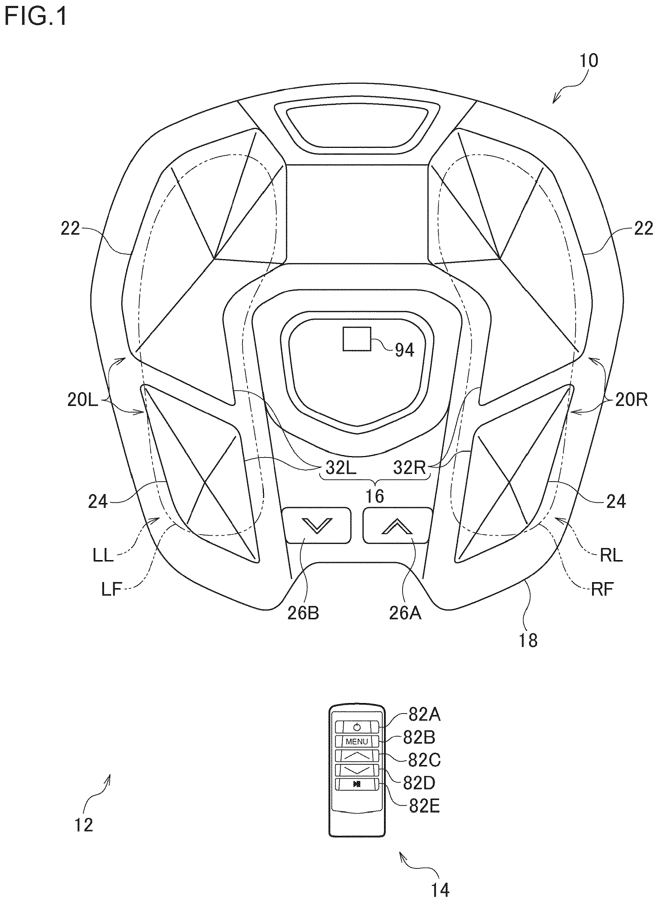

FIG. 1 is now referred to. An electrostimulation system 12 using an electrostimulation device 10 includes a remote controller 14 (hereinafter also referred to as "remote control 14") for operating the electrostimulation device 10 in addition to the electrostimulation device 10. The details of the remote control 14 will be described later.

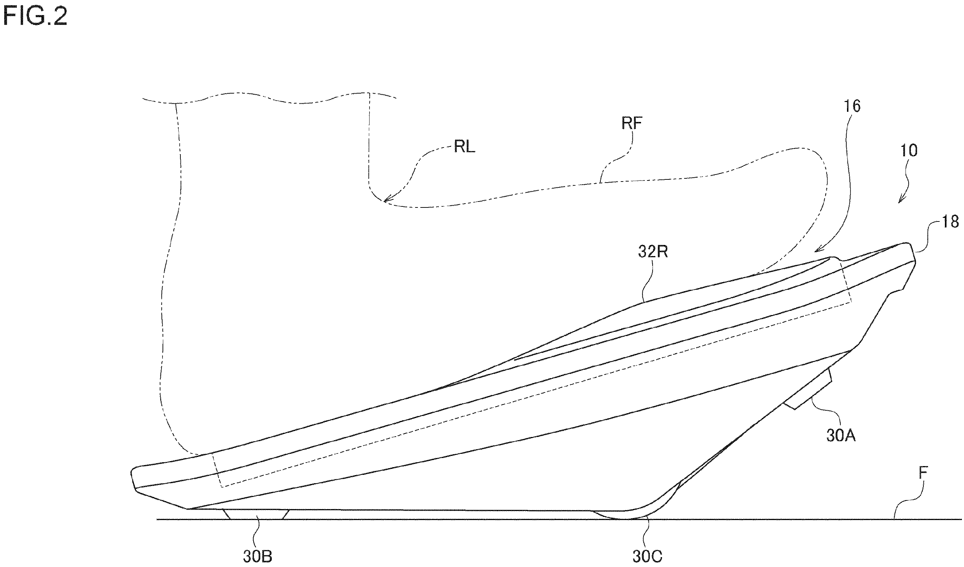

FIGS. 1 and 2 are now referred to. FIG. 1 is also a top view of the electrostimulation device 10. The electrostimulation device 10 includes an electrode group 16 that gives electrostimulation to both legs of the user and a main unit 18 in which the electrode group 16 is mounted.

The main unit 18 is used while being placed on a floor F. A pair of footrests 20L and 20R for placing the user's left and right feet are provided on an upper surface of the main unit 18. The pair of footrests 20L and 20R include a left footrest 20L for placing a left foot LF and a right footrest 20R for placing a right foot RF. The footrests 20L and 20R each include a front part 22 for placing a toe side part of a user's foot and a rear part 24 for placing a heel side part of the foot.

A plurality of device operation units 26A and 26B for operating the electrostimulation device 10 are provided on the upper surface of the main unit 18. The device operation units 26A and 26B are, for example, buttons that receive a pressing operation performed by the user. The device operation units 26A and 26B according to the present embodiment include a first level operation part 26A for raising the setting level of electrostimulation given to the user and a second level operation part 26B for lowering the setting level.

A plurality of grounding parts 30A-30C are provided on the lower surface of the main unit 18 at intervals in the front-back direction. The plurality of grounding parts 30A-30C include a front grounding part 30A provided on a front side of the main unit 18, a rear grounding part 30B provided on a rear side, and an intermediate grounding part 30C provided between the front grounding part 30A and the rear grounding part 30B. The main unit 18 can be swung back and forth with rotation around a left-right direction axis under a state in which the intermediate grounding part 30C is grounded.

The electrode group 16 includes a plurality of electrodes 32L and 32R for giving electrostimulation to both legs of the user. The plurality of electrodes 32L and 32R are individually provided respectively for the left and right legs. The plurality of electrodes 32L and 32R include a left electrode 32L corresponding to a left leg LL and a right electrode 32R corresponding to a right leg RL. The plurality of electrodes 32L and 32R are used while being in contact with the respectively corresponding legs LL and RL of the user. The left and right electrodes 32L and 32R according to the present embodiment constitute a pair of footrests 20L and 20R, respectively. More specifically, the left electrode 32L constitutes the left footrest 20L, and the right electrode 32R constitutes the right footrest 20R.

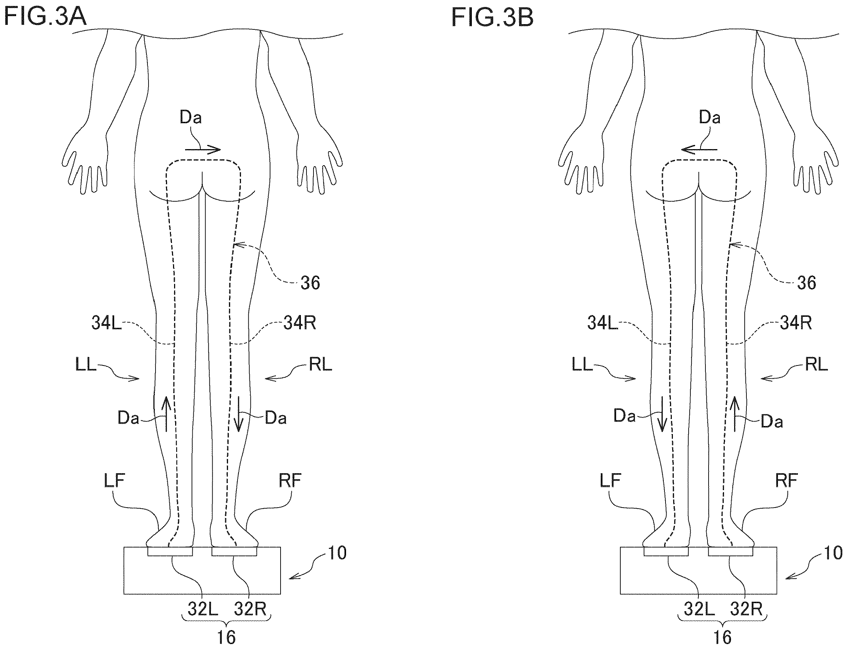

FIGS. 3A and 3B are now referred to. The electrode group 16 can form a left leg energizing path 34L for giving electrostimulation to the left leg LF and a right leg energizing path 34R for giving electrostimulation to the right leg RL when the plurality of electrodes 32L and 32R that constitute the electrode group 16 are in contact with the legs LL and RL. The electrode group 16 according to the present embodiment can form a both leg energizing path 36 that includes the left leg energizing path 34L and the right leg energizing path 34R. The left leg energizing path 34L includes at least a portion of the left leg LL. The left leg energizing path 34L includes the sole, lower leg, and upper leg of the left leg LF in the present embodiment. The right leg energizing path 34R includes at least a portion of the right leg RL. The right leg energizing path 34R includes the sole, lower leg, and upper leg of the right leg RL in the present embodiment. The both leg energizing path 36 includes the groin in addition to the left leg energizing path 34L and the right leg energizing path 34R.

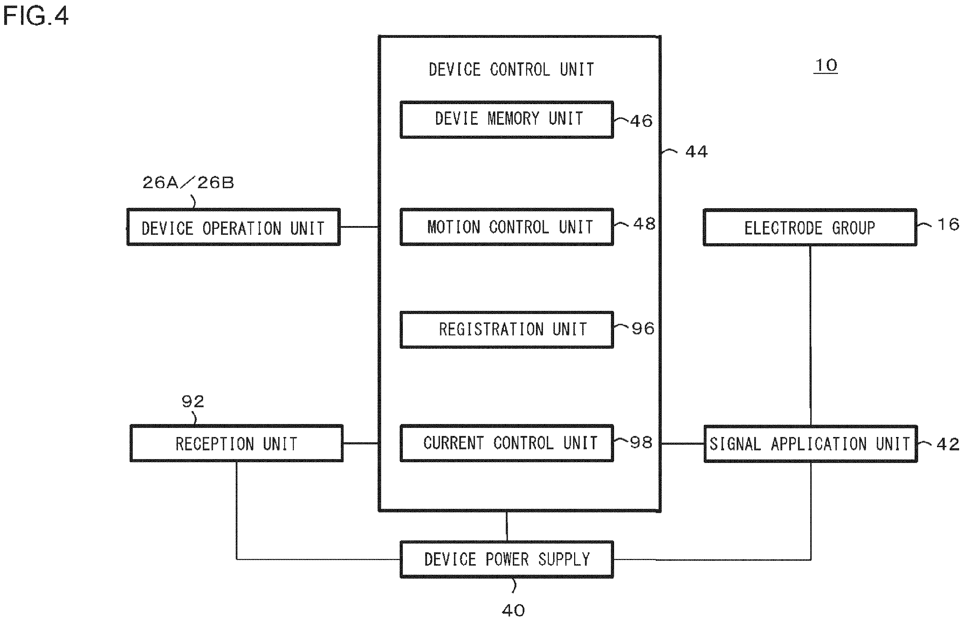

FIG. 4 is now referred to. Each block shown in the block diagram in the present specification can be implemented by electronic components, circuits, mechanical devices, etc., including central processing unit (CPU), read only memory (ROM), random access memory (RAM), etc., in terms of hardware, and by computer programs, etc., in terms of software. The figure illustrates functional blocks implemented by the cooperation of those components. It will be appreciated to a skilled person that these functional blocks may be implemented in a variety of forms by a combination of hardware and software.

In addition to the electrode group 16 and the device operation units 26A and 26B described above, the electrostimulation device 10 includes a device power supply 40, a signal application unit 42 that applies voltage signals to the electrode group 16, and a device control unit 44 (control device) that controls the electrostimulation device 10.

The device power supply 40 is housed in the main unit 18. The device power supply 40 according to the present embodiment is a primary battery such as a manganese battery. Alternatively, the device power supply 40 may also be a rechargeable secondary battery such as a lithium ion battery. The device power supply 40 supplies electric power to the signal application unit 42, the device control unit 44, etc.

The signal application unit 42 is formed using a pulse width modulation circuit or the like.

The device control unit 44 functions as a computer of the electrostimulation device 10. The device control unit 44 includes a device memory unit 46 that stores various types of information related to the electrostimulation device 10. The device control unit 44 executes a program stored in the device memory unit 46 so as to thereby perform a process for realizing the functions of the device control unit 44 (motion control unit 48, current control unit 98, etc., described below).

The device control unit 44 includes a motion control unit 48 that executes an electrostimulation motion to give electrostimulation by controlling the signal application unit 42. The electrostimulation motion is executed by applying an electrical muscle stimulation (EMS) waveform group for giving electrostimulation to the electrode group 16 as voltage signals by the signal application unit 42. The functions of the motion control unit 48 described below are exerted by executing a muscle electrostimulation program for giving electrostimulation to a muscle.

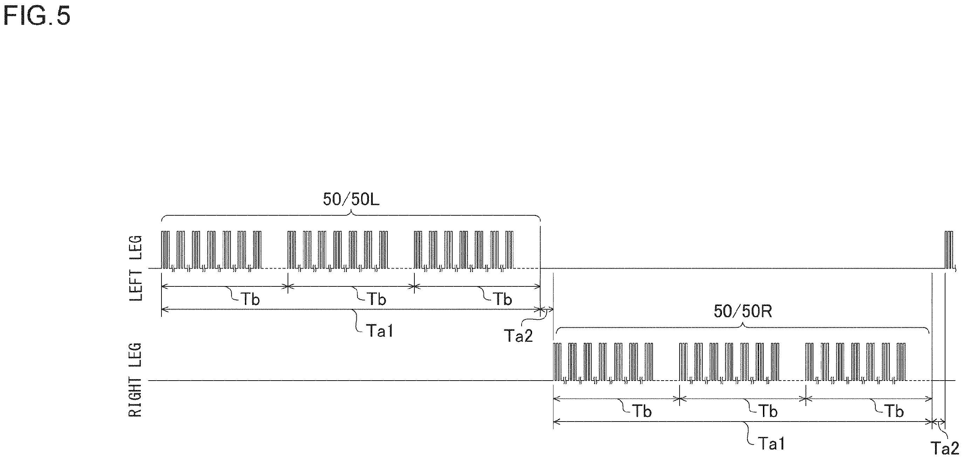

Fig. 5 is now referred to. The vertical axis in FIG. 5 indicates a voltage level. The EMS waveform group includes a left leg EMS waveform group 50L for giving electrostimulation to the left leg LL and a right leg EMS waveform group 50R for giving electrostimulation to the right leg RL. Hereinafter, when explaining a structure common to the left leg EMS waveform group 50L and the right leg EMS waveform group 50R, a term "EMS waveform group 50" collectively refers to these EMS waveform groups is used for the explanation. The EMS waveform group 50 is used to give electrostimulation equivalent to the walking motion of one step.

The device memory unit 46 stores waveform information that stores parameters (such as periods Ta1, Ta2, Tb, cycle Tf, cycle Tg, pulse output time Ti1, etc., as described below) related to the EMS waveform group 50. The waveform information is stored in the form of a table or the like. The motion control unit 48 can generate EMS waveform groups 50L and 50R having various waveforms by reading the waveform information stored in the device memory unit 46 and controlling the signal application unit 42 based on the read waveform information.

The motion control unit 48 can execute an alternating stimulation mode in which the left leg EMS waveform group 50L and the right leg EMS waveform group 50R are alternately applied to the electrode group 16 using the signal application unit 42. By executing the alternating stimulation mode, a stimulation current having the left leg EMS waveform group 50L and a stimulation current having the right leg EMS waveform group 50R are alternately output from the electrode group 16.

FIG. 3A shows a state in which a stimulation current having the left leg EMS waveform group 50L is being output from the electrode group 16, and FIG. 3B shows a state in which a stimulation current having the right leg EMS waveform group 50R is being output from the electrode group 16. In FIGS. 3A and 3B, the energization direction of the stimulation current is marked with an arrow Da.

When the both leg energizing path 36 is formed by the electrode group 16, the motion control unit 48 can give a perceptible level of electrostimulation only on either the left leg LL or the right leg RL by differentiating polarity of the electrodes 32L and 32R to which the EMS waveform groups 50L and 50R are applied according to the EMS waveform groups 50L and 50R to be applied. The details are described below.

The intensity of electrostimulation is the strongest at the point of input of the stimulation current having EMS waveform groups 50 L and 50 R and rapidly weakens away from the point of input. Therefore, when electrostimulation is given to the left leg LL, the point of input of the stimulation current needs to be the left electrode 32L. Therefore, the motion control unit 48 applies the left leg EMS waveform group 50L such that the left electrode 32L, which is the application destination, has positive polarity and the right electrode 32R has negative polarity. Thereby, a stimulation current having the left leg EMS waveform group 50L is energized from the left electrode 32L to the right electrode 32R in the both leg energizing path 36 (see FIG. 3A). As a result, a perceptible level of electrostimulation can be given to the left leg LL close to the left electrode 32L without applying a perceptible level of electrostimulation to the right leg RL. In the present embodiment, since the left electrode 32L constitutes the left footrest 20L, a perceptible level of electrostimulation is given to the sole of the left leg LL that is in contact with the left electrode 32L.

When electrostimulation is given to the right leg RL, the point of input of the stimulation current needs to be the right electrode 32R. Therefore, the motion control unit 48 applies the right leg EMS waveform group 50R such that the right electrode 32R, which is the application destination, has positive polarity and the left electrode 32L has negative polarity. Thereby, a stimulation current having the right leg EMS waveform group 50R is energized from the right electrode 32R to the left electrode 32L in the both leg energizing path 36 (see FIG. 3B). As a result, a perceptible level of electrostimulation can be given to the right leg RL close to the right electrode 32R without giving a perceptible level of electrostimulation to the left leg LL. In the present embodiment, since the right electrode 32R constitutes the right footrest 20R, a perceptible level of electrostimulation is given to the sole of the right leg RL that is in contact with the right electrode 32R.

The motion control unit 48 according to the present embodiment applies each of the left leg EMS waveform group 50L and the right leg EMS waveform group 50R in a stimulation giving period Ta1. The stimulation giving period Ta1 in the present embodiment consists of a plurality of consecutive block periods Tb. In the figure, the stimulation giving period Ta1 consisting of three consecutive block periods Tb is shown. The motion control unit 48 alternately applies the left leg EMS waveform group 50L and the right leg EMS waveform group 50R, with an interposed stimulation pause period Ta2 in which the applied voltage is zero.

A walking pace can be adjusted by changing the duration of the stimulation giving period Ta1 and the duration of the stimulation pause period Ta2. For example, as the duration of the stimulation giving period Ta1 and the duration of the stimulation pause period Ta2 are made longer, a walking motion at a slower walking pace can be reproduced. On the other hand, as these durations are made shorter, a walking motion at a faster walking pace can be reproduced. In alternately applying a left leg EMS waveform group 50L and a right leg EMS waveform group 50R, it is not essential to insert a stimulation pause period Ta2 between stimulation giving periods Ta1.

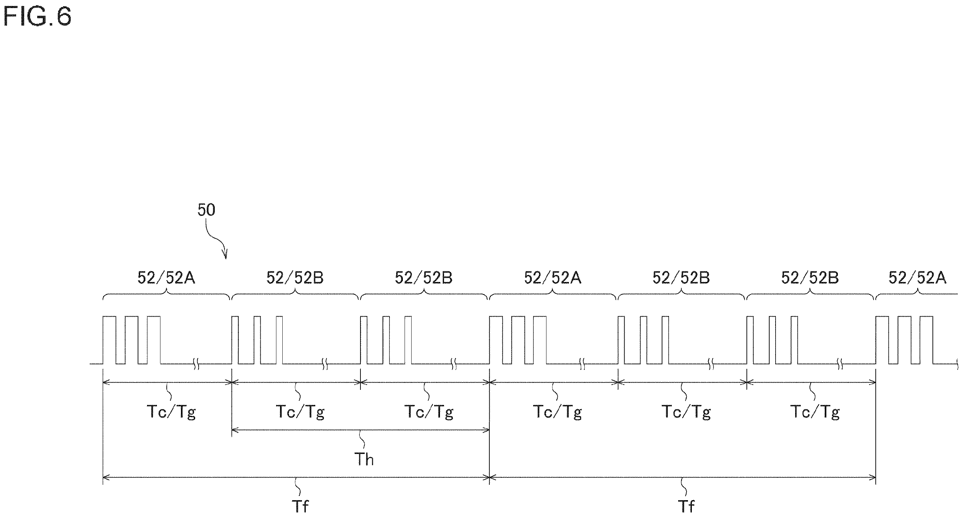

FIG. 6 is now referred to. FIG. 6 shows a part of an EMS waveform group 50 in one block period Tb of FIG. 5. The EMS waveform group 50 is formed by a plurality of pulse waveform groups 52 repeatedly applied to the electrode group 16. This means the EMS waveform group 50 includs a plurality of pulse waveform groups 52 repeatedly applied.

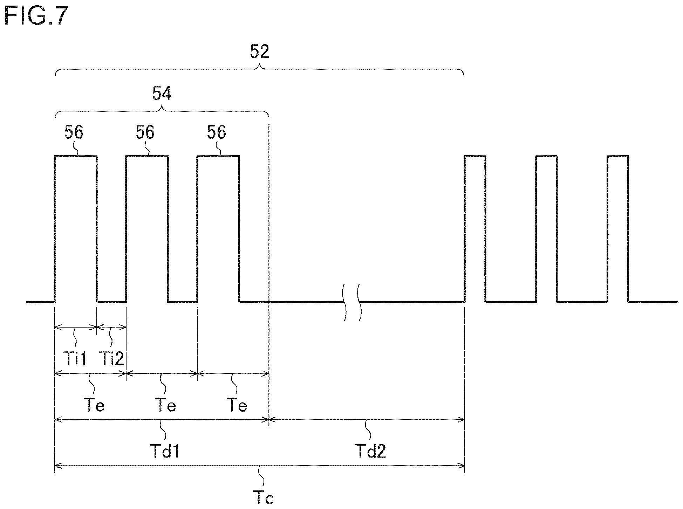

Fig. 7 is referred to. FIG. 7 shows a part of the pulse waveform group 52 of FIG. 6. An application period Tc of the pulse waveform group 52 is composed of a pulse continuation period Td1 during which a pulse train 54 is continuously applied and a pulse pause period Td2 during which the application of a pulse train 54 is suspended. A pulse train 54 consists of a plurality of pulse signals 56 of the same polarity with a predetermined number of pulses applied for each predetermined pulse cycle Te. The pulse voltage (voltage level) of the pulse signals 56 has a magnitude set by an input operation to the device operation units 26A and 26B.

The pulse continuation period Td1 and the pulse cycle Te are set to be very short. The pulse train 54 output in the pulse continuation period Td1 is recognized as a single electrostimulation. The duration of the pulse pause period Td2 is longer than that of the pulse continuation period Td1 in order to reduce user's pain caused by the electrostimulation. FIG. 7 shows an example where the pulse continuation period Td1 is 2.1 ms, the pulse pause period Td2 is 14.7 ms, the pulse cycle Te is 700 µs, and the number of pulses is 3.

FIG. 6 is now referred to. The plurality of pulse waveform groups 52 include a first pulse wave group 52A applied intermittently every first cycle Tf and a second pulse waveform group 52B applied outside an application period Tc of the first pulse waveform group 52A in the first cycle Tf. Outside the application period Tc of the first pulse waveform group 52A in this case refers to a period Th obtained by excluding the application period Tc of the first pulse waveform group from the first cycle Tf. FIG. 6 shows an example where one first pulse waveform group 52A and two second pulse waveform groups 52B are included within the first cycle Tf. The first pulse waveform group 52A and the second pulse waveform groups 52B share the same duration for the application period Tc, the pulse continuation period Td1, the pulse pause period Td2, and the pulse cycle Te.

The pulse waveform groups 52 according to the present embodiment are repeatedly applied every second cycle Tg, which is shorter than the first cycle Tf, within the same first cycle Tf. The part for one second cycle Tg in the first cycle Tf serves as an application period Tc for a first pulse waveform group 52A, and the part for the remaining second cycles Tg in the first cycle serves as application periods Tc for second pulse waveform groups 52B. The second cycle Tg is a duration obtained by dividing the first cycle Tf equally by N + 1 when the number of the second pulse waveform groups 52B in the first cycle Tf is denoted as N.

First pulse waveform groups 52A are mainly used to promote incomplete tetanic contraction to muscles. In relation to this purpose, the first cycle Tf is set to be in the range of 40 ms to 66 ms corresponding to a frequency of 15 Hz to 25 Hz, which can promote incomplete tetanic contraction to the muscles. FIG. 6 shows an example where the first cycle Tf is 50.00 ms and the second cycle Tg is 16.66 ms (= 50.00/(2+1)). The frequency corresponding to the first cycle Tf is preferably 18 Hz to 23 Hz, more preferably 19 Hz to 21 Hz.

When only a first pulse waveform group 52A is applied every first cycle Tf, wavy electrostimulation is intermittently given to the user. If such intermittent electrostimulation is given to the legs, the physical sensation of continuously stepping on the ground cannot be reproduced. As a countermeasure, the motion control unit 48 applies a second pulse waveform group 52B between consecutive first pulse waveform groups 52A. This allows continuous electrostimulation to be given to the user compared to a case where only a first pulse waveform group 52A is applied every first cycle Tf. When such a pulse waveform group 52 is applied to the legs, it is possible to reproduce the physical sensation of continuously stepping on the ground.

By changing the pulse waveforms of the first pulse waveform groups 52A and the second pulse waveform groups 52B described above, the physical sensation that can be given to the user can be changed in various ways. The "pulse waveforms" in this case refer to the waveforms of pulse signals 56 in the pulse train 54.

For example, by making a pulse output time Ti1 (see FIG. 7) of each pulse signal 56 of a first pulse waveform group 52A longer than a pulse output time Ti1 of each pulse signal 56 of a second pulse waveform group 52B, the physical sensation given by the first pulse waveform group 52A can be made stronger. This is synonymous with making the duty ratio of the pulse signal 56 of the first pulse waveform group 52A to be larger than the duty ratio (ratio of the pulse output time Ti1 to the pulse cycle Te) of the pulse signal 56 of the second pulse waveform group 52B. By making the physical sensation caused by the first pulse waveform group 52A with a frequency of 15 Hz to 25 Hz to be strong, it becomes easier to induce incomplete tetanic contraction in the muscles. Therefore, while the user is given a physical sensation of receiving continuous electrostimulation, muscle fatigue can be easily induced. When such a pulse waveform group 52 is applied to the legs, it is possible to induce muscle fatigue while giving a physical sensation of continuously stepping on the ground. In relation to such effects, when the stimulation giving period Ta1 includes multiple block periods Tb, at least one block period Tb needs to satisfy the conditions related to this pulse output time Ti1.

In contrast, the shorter the pulse output time Ti1 of the first pulse waveform group 52A relative to the pulse output time Ti1 of the second pulse waveform group 52B, the weaker the physical sensation given by the first pulse waveform group 52A. If the pulse output time Ti1 of the second pulse waveform group 52B and the pulse output time Ti1 of the first pulse waveform group 52A are the same, the pulse waveform group 52 is being applied for each frequency exceeding 25 Hz. By making the physical sensation caused by the first pulse waveform group 52A with a frequency of 15 Hz to 25 Hz to be weak, fatigue to the muscles can be reduced. Therefore, muscle fatigue can be moderately suppressed while giving the user a physical sensation of receiving continuous electrostimulation. When such a pulse waveform group 52 is applied to the legs, it is possible to moderately suppress muscle fatigue while giving a physical sensation of continuously stepping on the ground.

The effects explained thus far can be obtained in the same manner when the pulse voltage (voltage level) of the pulse signal 56 of the first pulse waveform group 52A is set higher than the pulse voltage of the pulse signal 56 of the second pulse waveform group 52B. Further, in obtaining the effects explained thus far, the pulse output time Ti1 of the first pulse waveform group 52A may be set to be longer than the pulse output time Ti1 of the second pulse waveform group 52B, and the pulse voltage of the first pulse waveform group 52A may be set to be higher than the pulse voltage of the second pulse waveform group 52B.

Fig. 5 is now referred to. Each of the plurality of block periods Tb is different in at least one of the pulse waveform of a pulse waveform group 52 applied in the block period Tb or the duration of the block period Tb. For example, if the pulse output time Ti1 of a first pulse waveform group 52A is gradually increased as the number of subsequent block periods Tb increases, the intensity of the electrostimulation given to the user can be gradually increased. In contrast, if the pulse output time Ti1 of a first pulse waveform group 52A is gradually decreased as the number of subsequent block periods Tb increases, the intensity of the electrostimulation given to the user can be gradually decreased. By increasing or decreasing the duration of each block period Tb, the length of the physical sensation according to each block period Tb can also be increased or decreased. As explained below, this allows the intensity of the electrostimulation given to the user to be changed in stages, and the diversification of physical sensations given to the user can be achieved. In any block period Tb, the first cycle Tf and the second cycle Tg for each pulse waveform group 52 have the same duration.

Next, various motion modes using the alternating stimulation mode described above will be explained. The motion control unit 48 starts executing the following motion modes when predetermined start conditions are met. The start conditions include, for example, receiving a predetermined input operation to the electrostimulation device 10 or the remote control 14. The motion control unit 48 may execute at least the motion modes explained below individually or may execute each motion mode in sequence.

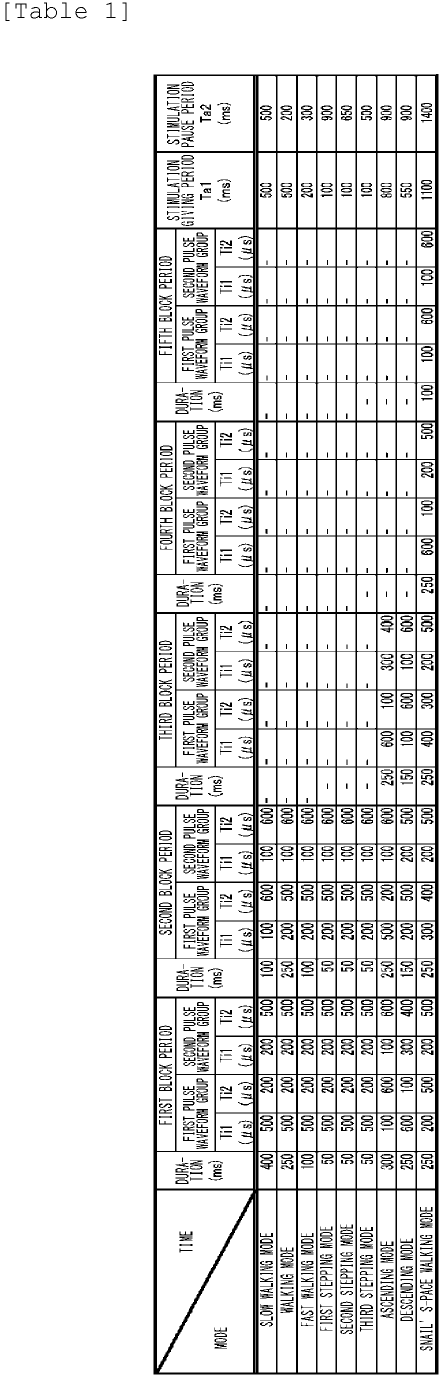

Table 1 shows the timetables for the various motion modes using the alternating stimulation mode. Ti2 in Table 1 is a pulse off period in the pulse cycle Te (see also FIG. 7). A walking mode is a mode that reproduces the normal walking motion. In this case, a slow walking mode, a walking mode, and a fast walking mode are shown as walking modes. In each walking mode, the walking pace given as a physical sensation is changed by changing the length of the stimulation giving period Ta1 and the length of the stimulation pause period Ta2. For example, the total amount of time of the stimulation giving period Ta1 and the stimulation pause period Ta2 gradually shortens in the order of the slow walking mode, the walking mode, and the fast walking mode. This indicates that the walking pace given as a physical sensation becomes faster in the order of the slow walking mode, the walking mode, and the fast walking mode. Further, in each walking mode, the pulse output time Ti1 of the first pulse waveform group 52A is reduced in the order of the first block period Tb and the second block period Tb, and the electrostimulation is weakened in stages. This reproduces the motion in which the stimulation given to the feet becomes weaker as the ground contact area increases in the walking motion.

A stepping mode is a mode that reproduces stepping. Here, the first, second, and third stepping modes are shown as stepping modes. In each stepping mode, the stimulation giving period Ta1 is shorter than that of the walking mode in order to give a physical sensation of rhythmically and lightly stepping with feet. In each stepping mode, the stepping pace given as a physical sensation is changed by changing the total amount of time of the stimulation giving period Ta1 and the stimulation pause period Ta2. For example, the total amount of time of the stimulation giving period Ta1 and the stimulation pause period Ta2 gradually shortens in the order of the first stepping mode, the second stepping mode, and the third stepping mode. This indicates that the stepping pace given as a physical sensation becomes faster in the order of the first stepping mode, the second stepping mode, and the third stepping mode.

An ascending mode is a mode that reproduces the walking motion when going up stairs. In the ascending mode, the stimulation giving period Ta1 consists of three block periods Tb. In the ascending mode, the pulse output time Ti1 of the first pulse waveform group 52A is increased in the order of the first block period Tb, the second block period Tb, and the third block period Tb. This means that in the ascending mode, the electrostimulation given as a physical sensation becomes gradually stronger. This reproduces a state where the stimulation applied to one foot is weak when the foot lands on the ground because the weight is applied to both feet when going up stairs and the stimulation becomes stronger because all the weight is applied to the foot when kicking the other foot against the ground.

A descending mode is a mode that reproduces the walking motion when going down the stairs. In the descending mode, the stimulation giving period Ta1 consists of the three block periods Tb. In the descending mode, the pulse output time Ti1 of the first pulse waveform group 52A is decreased in the order of the first block period Tb, the second block period Tb, and the third block period Tb. This means that in the descending mode, the electrostimulation given as a physical sensation becomes gradually weaker. This reproduces a state where when going down the stairs, the stimulation is strong because one foot lands vigorously with the weight on the foot at the time of landing the ground, and the stimulation becomes weak since the load is distributed to the entire foot after a while.

A snail's-pace walking mode is a mode that reproduces a slow and powerful walking motion. The stimulation giving period Ta1 of the snail's-pace walking mode consists of five block periods Tb. In the snail's-pace walking mode, the pulse output time Ti1 of the first pulse waveform group 52A is gradually increased from the first block period Tb to the fourth block period Tb and is decreased in the fifth block period Tb. In the snail's-pace walking mode, the stimulation giving period Ta1 is longer than that in the walking mode. This means that in the snail's-pace walking mode, the electrostimulation given as a physical sensation slowly and gradually becomes larger and then weakens at once. This reproduces a motion of gradually increasing the force of stepping on the ground and slowly lifting the foot off the ground at the end.

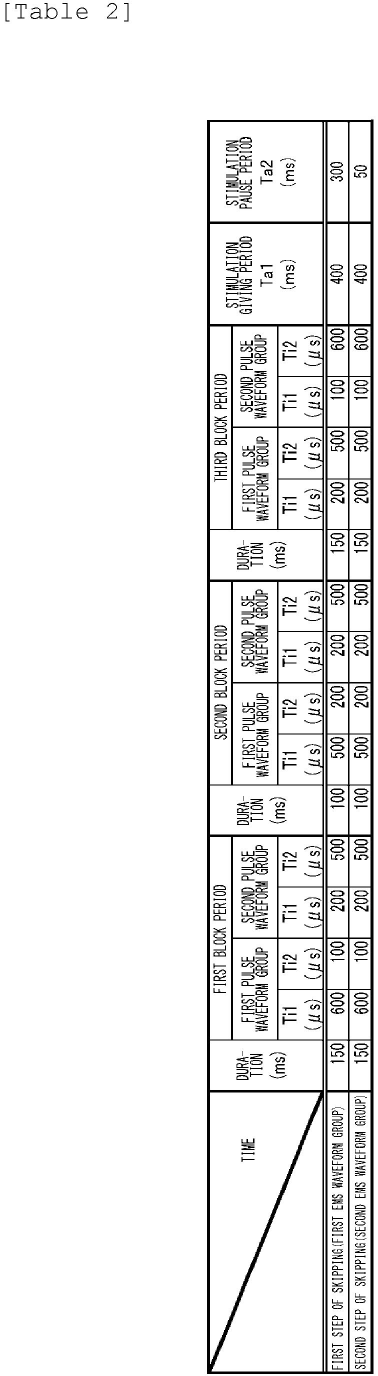

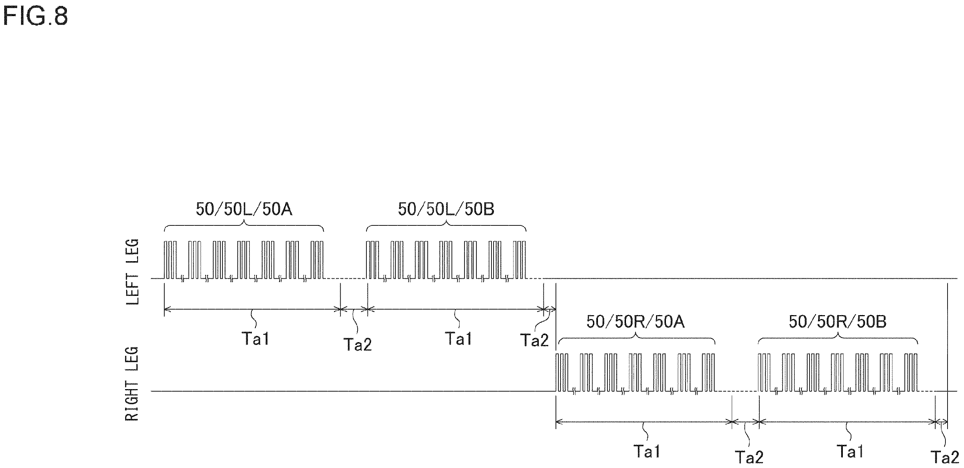

FIG. 8 is also referred to. A skip mode is a mode that reproduces skipping motion. In a step mode, a plurality of intermittent left leg EMS waveform groups 50L and a plurality of intermittent right leg EMS waveform groups 50R are alternately applied to the electrode group 16. The plurality of left leg and right leg EMS waveform groups 50L and 50R include a first EMS waveform group 50A corresponding to a first step walking motion and a second EMS waveform group 50B corresponding to a second step walking motion by the same leg as the first step walking motion. Table 2 shows a timetable for the first EMS waveform group 50A and the second EMS waveform group 50B applied in the skip mode. Between the first EMS waveform group 50A and the second EMS waveform group 50B, there is a stimulation pause period Ta2 during which the applied voltage is zero. Thereby, intermittent electrostimulation is repeatedly given to the same leg, and a skipping motion in which the same leg continuously bounces is repeated with both legs can be reproduced.

The effect of the above ingenuity will be now explained.

The plurality of pulse waveform groups 52 include a first pulse wave group 52A applied intermittently every first cycle Tf corresponding to a frequency of 15 Hz to 25 Hz and a second pulse waveform group 52B applied outside of an application period of the first pulse waveform group 52A in the first cycle Tf. Therefore, by changing the pulse waveforms of the first pulse waveform group 52A and the second pulse waveform group 52B, the physical sensation that can be given to the user can be easily changed as described above. Consequently, it is possible to diversify the physical sensations that can be given to the user.

The pulse signal of the first pulse waveform group 52A of 15 Hz to 25 Hz has a larger pulse output time Ti1 or pulse voltage than the pulse signal 56 of the second pulse waveform group 52B. This allows a moderately smaller load to be applied to the user compared to a case where only the first pulse waveform group 52A of 15 Hz to 25 Hz is applied. This is a finding obtained through experimental studies conducted by the inventors of the present application, as explained below. Therefore, by providing the user with a physical sensation that is difficult to become bored with, the user can be given a moderate sense of fatigue while being encouraged to continue using the electrostimulation device 10. As a result, compared to a case where only the first pulse waveform group 52A with a frequency of 15 Hz to 25 Hz is output, a physical sensation suitable for maintaining muscle mass can be given.

The electrostimulation device 10 includes a motion control unit 48 that applies the left leg EMS waveform group 50L and the right leg EMS waveform group 50R alternately to the electrode group 16 by controlling the signal application unit 42. Thereby, the left leg EMS waveform group 50L and the right leg EMS waveform group 50R are alternately output from the electrode group 16, allowing an unprecedented physical sensation to be given to the user that makes him/her feel as if he/she were walking. In other words, it is possible to provide the user with a physical sensation that is difficult to become bored with. Consequently, this makes it easier to encourage continued use of the electrostimulation device 10.

Next, an explanation will be given regarding a test conducted in order to check the effects of the alternating stimulation mode described above.

A total of twelve subjects were asked to use the walking mode explained below on a one-time basis using the aforementioned electrostimulation device 10. The subjects consisted of six males and six females, with ages in the range of 68.58 ± 3.53 years. The voltage level (pulse voltage) of the electrostimulation device 10 was set to the maximum level the subjects could tolerate.

The walking mode refers to a mode of a total of 20 minutes that is performed in the following order: a stepping mode (three minutes), a pause mode (30 seconds), a snail's-pace walking mode (three minutes), a walking mode (three minutes), a pause mode (30 seconds), an ascending mode (three minutes), a descending mode (three minutes), a skip mode (three minutes), and a pause mode (one minute). The stepping mode in this case was executed in the order of the first stepping mode (one minute), the second stepping mode (one minute), and the third stepping mode (one minute) described above. The pause mode is a mode where the application of an EMS waveform group is paused.

Along with this, the same subjects were asked to use a training mode explained next, using the aforementioned electrostimulation device 10. This training mode is a mode where a pulse waveform group is output at a frequency (= 20Hz) suitable for promoting incomplete tetanic contraction for a total of 23 minutes.

In order to evaluate the subject's muscle fatigue using the electrostimulation device 10, the muscle fatigue measurement explained next was performed. In the muscle fatigue measurement, the subjects' muscle strength was measured using a simple muscle strength measuring device (Mutus F-1 manufactured by ANIMA Corporation). In this muscle fatigue measurement, muscle strength immediately before the use of the electrostimulation device 10 and muscle strength immediately after the use of the electrostimulation device 10 were measured. For the muscle strength immediately after the use of the electrostimulation device 10, the muscle strength immediately after the use of the walking mode and the muscle strength immediately after the use of the training mode were measured separately.

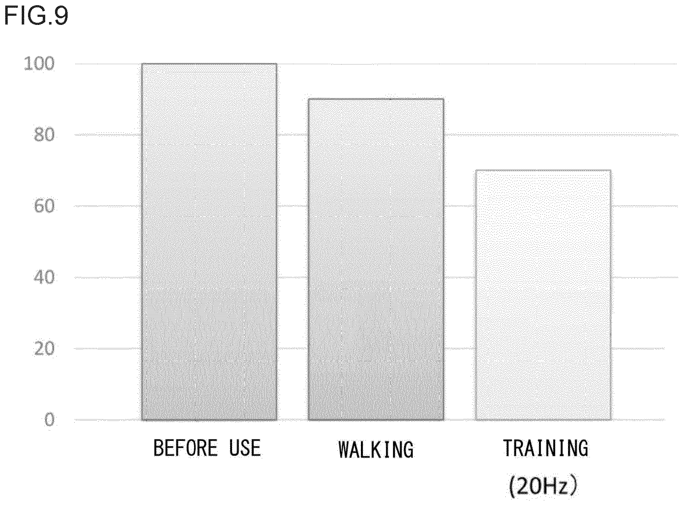

FIG. 9 is now referred to. The figure shows the ratio of the muscle strength immediately after the use to the muscle strength before the use given that the muscle strength immediately before the use of the electrostimulation device 10 is set to be 100. The results were obtained showing that when the walking mode was used, the muscle strength decreased by 10% on average compared to that before the use of the walking mode. Further, it can be understood that when the walking mode was used, the degree of decrease in muscle strength was smaller than that when the training mode was used. This confirms that when the walking mode is used, the exercise load is lighter than that when the training mode is used for the purpose of muscle hypertrophy and that this mode is suitable for the maintenance of muscle mass.

Next, a total of six subjects were asked to continuously use the aforementioned walking mode using the aforementioned electrostimulation device 10. The subjects consisted of three males and three females, with ages in the range of 69.83 ± 2.71 years. The voltage level (pulse voltage) of the electrostimulation device 10 was set to the maximum level the subjects could tolerate. The subjects were asked to use the walking mode once daily for a total of 20 minutes during the test period, which was a total of four weeks.

In order to evaluate the gastrocnemius muscle strength of the subjects, the gastrocnemius muscle strength measurement explained below was performed. In the gastrocnemius muscle strength measurement, the subjects' muscle strength was measured using the simple muscle strength measuring device (Mutus F-1 manufactured by ANIMA Corporation). In this muscle fatigue measurement, muscle strength immediately before the test period and muscle strength immediately after the test period were measured.

Further, in order to evaluate the subjects' walking speed, walking speed measurement explained below was performed. In the walking speed measurement, the time required to walk five meters at normal walking speed and the time required to walk five meters at maximum walking speed were measured using a stopwatch. The normal walking speed is the walking speed at which a subject normally walks, and the maximum walking speed is the speed at which the subject walks as fast as possible without running.

In order to evaluate the subject's two-step stride length, a two-step test explained below was performed. In the two-step test, the subject's stride length was measured using a tape measure when the subject walked for two steps.

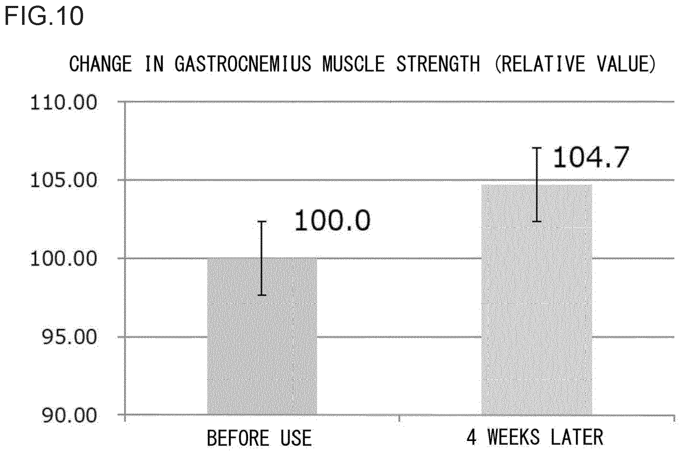

FIG. 10 is referred to. The figure shows the ratio of the muscle strength immediately after the test period to the muscle strength immediately before the test period given that the muscle strength immediately before the test period is set to be 100. Continuous use of the walking mode resulted in an average increase of 4.7% in muscle strength compared to that immediately before the test period.

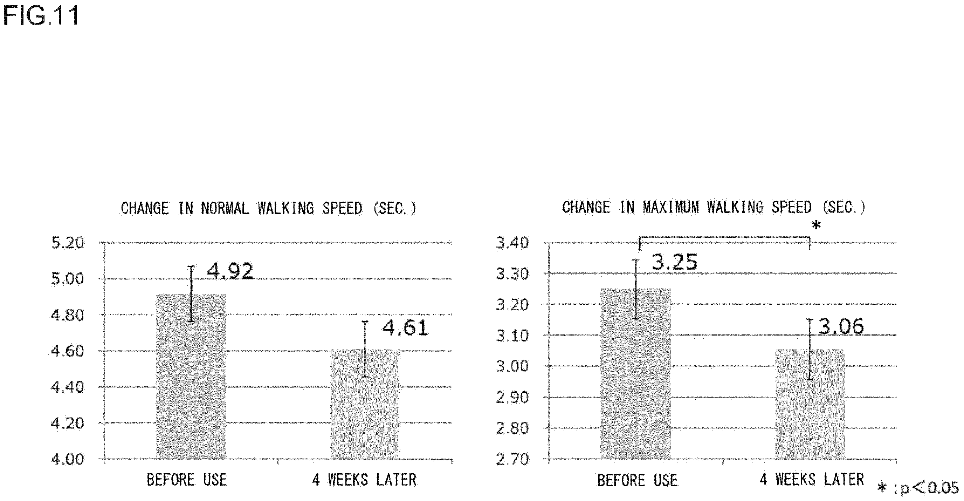

FIG. 11 is referred to. Continuous use of the walking mode resulted in an average increase of about 0.31 seconds at normal walking speed compared to that immediately before the test period. Also, compared to immediately before the test period, on average, the maximum walking speed increased by 0.19 seconds, and there was a significant difference (p < 0.05).

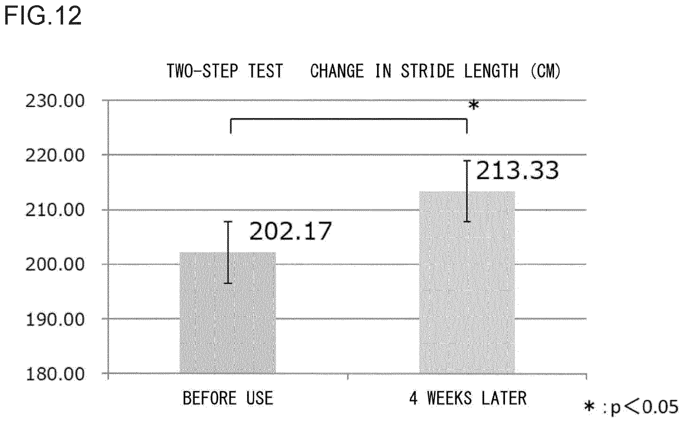

FIG. 12 is referred to. Continuous use of the walking mode resulted in an average increase of about 11.16 cm in stride length compared to that immediately before the test period, and there was a significant difference (p < 0.05). Both test results provide support for the fact that the walking mode can be used to moderately reduce the exercise load on the user.

Next, other ingenuity for the electrostimulation system 12 will be now explained.

A situation is assumed where multiple users use individual electrostimulation devices 10 simultaneously. This can be realized, for example, in a gym, a nursing home, a rehabilitation room, etc. In this case, one user's remote control 14 may accidentally operate another user's electrostimulation device 10.

As a countermeasure, a policy can be conceivable where only when the electrostimulation device 10 receives a preset version of command data, the electrostimulation device 10 is operated based on the command data. In this case, it is necessary to set the version of the command data to be output from among multiple versions in the remote control 14. In the same way, it is also necessary to set the version of the command data to be received from among multiple versions in the electrostimulation device 10. Therefore, it is necessary to set the version to be used for both the remote control 14 and the electrostimulation device 10.

The version of the command data is usually switched by a switching operation of sliding a slide switch provided on the remote control 14 or the electrostimulation device 10, simultaneously pressing multiple buttons, or the like. The number of versions that can be switched by such switching operations is about two or three at most. Therefore, it is not suitable for use in situations where more users use the electrostimulation device 10 at the same time.

The following is an explanation regarding ingenuity that used in order to prevent the occurrence of wrong operation in such situations where multiple users use the electrostimulation device 10 at the same time.

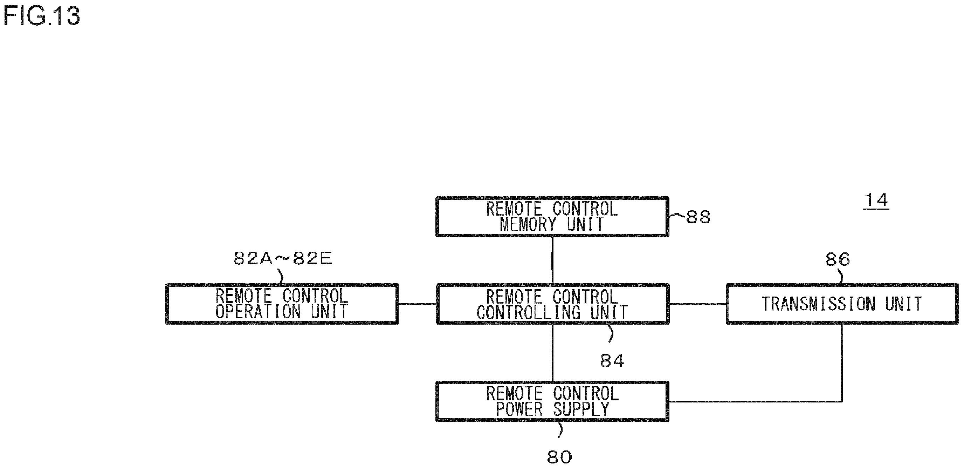

FIG. 13 is referred to. The remote control 14 includes a remote control power supply 80, a plurality of remote control operation units 82A-82E that receive input operations by users, a remote control controlling unit 84 that controls the remote control 14, a transmission unit 86 that can transmit transmission signals using infrared rays or the like, and a remote control memory unit 88.

The remote control power supply 80 is housed in the housing of the remote control 14. The remote control power supply 80 is a primary battery, secondary battery, or the like. The remote control power supply 80 supplies electric power to the remote control controlling unit 84, the transmission unit 86, etc.

The plurality of remote control operation units 82A-82E include a power supply operation unit 82A for turning the power on and off, a menu operation unit 82B for selecting a mode, level operation units 82C and 82D for changing the setting level, and a switching operation unit 82E for pausing or playing back the motion of the electrostimulation device 10. The level operation units 82C and 82D include a first level operation unit 82C for raising the setting level and a second level operation unit 82D for lowering the setting level.

The remote control memory unit 88 stores identification information unique to the remote control 14. The identification information is a serial code or the like. The identification information is composed of, for example, a random number generated by a random number generator incorporated in the remote control 14. The random number generator generates a random number with a predetermined number of bits (e.g., 16 bits). In addition, the identification information may also be written to the remote control memory unit 88 through an input operation to the remote control operator.

The remote control controlling unit 84 generates command data corresponding to input operations to the remote control operation units 82A-82E. The command data is for instructing the operation of the electrostimulation device 10. The remote control controlling unit 84 transmits a transmission signal including the generated command data to the electrostimulation device 10 through the transmission unit 86.

FIG. 4 is now referred to. The electrostimulation device 10 includes a reception unit 92 that receives the transmission signal transmitted from the transmission unit 86 of the remote control 14. The reception unit 92 receives a transmission signal transmitted through the receiving window 94 (see FIG. 1) provided in the main unit 18.

The device control unit 44 of the electrostimulation device 10 includes a registration unit 96 that registers the identification information of the remote control 14 to be paired with the electrostimulation device 10 in the device memory unit 46. The registration unit 96 executes a registration process (pairing process) of registering the remote control 14 when a predetermined registration process start condition is met. The registration process start condition is to perform a predetermined input operation on the device operation units 26A and 26B of the electrostimulation device 10 and the remote control operation units 82A to 82E of the remote control 14. The input operation to the remote control 14 is, for example, simultaneous pressing of the power supply control unit 82A and menu operation unit 82B of the remote control 14, i.e., performing a simultaneous input operation of the plurality of remote control operation units 82A to 82E. This prevents a situation where the registration process is accidentally executed while the remote control 14 is in use. The input operation to the electrostimulation device 10 is, for example, to press the level operation unit 26A of the electrostimulation device 10.

When an input operation for the registration process is performed on the remote control 14, the remote control controlling unit 84 of the remote control 14 generates a processing code for the registration process and transmits the processing code from the transmission unit 86. Identification information unique to the remote control 14, which is stored in the remote control memory unit 88 of the remote control 14, is added to the processing code. When the processing code transmitted from the remote control 14 is received by the reception unit 92 of the electrostimulation device 10 under a condition where an input operation for the registration process for the electrostimulation device 10 has been performed, the registration unit 96 registers the identification information added to the processing code in the device memory unit 46 as identification information unique to the remote control 14. This completes the registration process.

When the remote control controlling unit 84 of the remote control 14 transmits a transmission signal containing command data, the identification information stored in the remote control memory unit 88 is added to the command data. The command data is set according to a predetermined format, and the identification information is stored at a predetermined position within that format. The motion control unit 48 of the electrostimulation device 10 performs the motion according to the command data only when the motion control unit 48 receives the command data to which the identification information corresponding to the registered remote control 14 is added in the device memory unit 46. Thereby, even in a situation where multiple users use electrostimulation devices 10 at the same time, only when command data transmitted from the remote control 14 registered in the electrostimulation device 10 is received, the motion according to the command data can be performed. Eventually, in a situation where multiple users use electrostimulation devices 10 at the same time, the occurrence of mistakenly operating another user's electrostimulation device can be prevented.

A case is now considered where a repeat command for causing the electrostimulation device 10 to repeat a predetermined motion is transmitted. In this case, the remote control controlling unit 84 of the remote control 14 may transmit a repeat command with the identification information stored in the remote control memory unit 88. In addition to this, the repeat command does not need to be used in repeating a predetermined motion. In this case, the identification information may be added to a command corresponding to a motion to be repeated, and the command with the identification information may be transmitted repeatedly.

Next, other ingenuity for the electrostimulation device 10 will be now explained.

When a commercial power source is used as the device power supply 40, since electric power can be constantly supplied, it is common to maintain the reception unit 92 of the electrostimulation device 10 in a receivable state at all times. In contrast, when a battery is used as the device power supply 40, maintaining the reception unit 92 of the electrostimulation device 10 in a receivable state at all times causes the battery to consume a large amount of power when the electrostimulation device 10 is not in use. This results in a problem that the actual usable time of the electrostimulation device 10 is shortened.

Taking this into account, the following is an explanation of ingenuity for reducing power consumption in the electrostimulation device 10.

The device control unit 44 of the electrostimulation device 10 includes a current control unit 98 that controls the electric current supplied to the reception unit 92. The current control unit 98 can cause the reception unit 92 to perform an intermittent reception mode in which the reception unit 92 is maintained intermittently in a receivable standby state and a constant reception mode in which the reception unit 92 is maintained in a receivable standby state at all times. The current control unit 98 can cause the reception unit 92 to perform the intermittent reception mode by intermittently supplying a current of a predetermined set value to the reception unit 92. Further, the current control unit 98 can cause the reception unit 92 to perform the constant reception mode by constantly supplying a current of a predetermined set value to the reception unit 92.

When being in the intermittent reception mode, the current control unit 98 intermittently supplies a current of a predetermined set value to the reception unit 92 for a predetermined reception period (e.g., 2 ms) every predetermined intermittent cycle (e.g., 200 ms). When being in the intermittent reception mode, the current control unit 98 suppresses power consumption by limiting the magnitude of the current supplied to the reception unit 92 outside the reception period to be equal to or less than a limit value that is smaller than the set value.

When a transmission signal is received by the reception unit 92 in the intermittent reception mode, the current control unit 98 extends the reception period during which signals can be received by the reception unit 92. When the current control unit 98 receives a transmission signal containing a predetermined command from the remote control 14 during this extended reception period, the current control unit 98 shifts from the intermittent reception mode to the constant reception mode. This predetermined command is, for example, a power-on command to turn on the device power supply 40 of the electrostimulation device 10. In contrast, when receiving any other transmission signal during this extended reception period, the current control unit 98 limits the current supplied to the reception unit 92 to be equal to or less than the limit value and continues the intermittent reception mode.

When being in the constant reception mode, the current control unit 98 shifts from the constant reception mode to the intermittent reception mode when a predetermined end condition (e.g., a predetermined time elapses after the end of the reception of a transmitted signal) is met.

As described above, by executing the intermittent reception mode, the current control unit 98 can suppress the power consumption by the reception unit 92 more compared to a case where the reception unit 92 is maintained in the constant reception mode.

When transmitting transmission signals containing a power-on command, the transmission unit 86 of the remote control 14 repeatedly transmits transmission signals for a predetermined number of repetitions (e.g., six) such that a predetermined transmission period and a transmission interval period appear alternately. When the current control unit 98 receives transmission signals containing a power-on command continuously, the current control unit 98 may ignore the second and subsequent transmission signals. Receiving signals continuously in this case means when an interval period between the last received transmission signal and a newly received transmission signal is the aforementioned transmission interval period plus or minus extra.

Other variations of each component are explained below.

It is only necessary for the electrode group 16 to be able to give electrostimulation to both legs LL and RL of the user by forming at least the left leg energizing paths 34L and 34R, and the specific examples thereof are not limited. The right side electrode 32R and left side electrode 32L of the electrode group 16 may be provided in an attachment tool such as a belt separate from the main unit 18 having the footrests 20L and 20R, for example. Alternatively, the left side electrode 32L may be provided individually in an attachment tool for the left leg LL and in the main unit 18, and the right side electrode 32R may be provided individually in an attachment tool for the right leg RL and in the main unit 18.

An example has been explained where the electrode group 16 can form both leg energizing path 36 that includes the left leg energizing path 34L and the right leg energizing path 34R. Alternatively, the electrode group 16 may form a left leg energizing path 34L and a right leg energizing path 34R that are individually separated. This is based on the assumption that a plurality of left side electrodes 32L form the left leg energizing path 34L and a plurality of right side electrodes 32R form the right leg energizing path 34R. In detail, this is a case where the left side electrodes 32L and the right side electrodes 32R are provided in the left and right attachment tools and the main unit 18 described above.

In the case of forming such a leg energizing path 36 and a right leg energizing path 34R that are separate, the motion control unit 48 may apply different combinations of electrodes 32L and 32R, which are the application destinations, according to the EMS waveform groups 50L and 50R to be applied in order to give a perceptible level of electrostimulation only on either the left leg LL or the right leg RL. More specifically, when applying the left leg EMS waveform group 50L, the motion control unit 48 needs to apply the left leg EMS waveform group 50L only to the plurality of left side electrodes 32L forming the left leg energizing path 34L. Thereby, a stimulation current having the left leg EMS waveform group 50L is energized only on the left leg energizing path 34L, and a perceptible level of electrostimulation only by the left leg LL is given. Further, when applying the right leg EMS waveform group 50R, the motion control unit 48 needs to apply the right leg EMS waveform group 50R only to the plurality of right side electrodes 32R forming the right leg energizing path 34R. Thereby, a stimulation current having the right leg EMS waveform group 50R is energized only on the right leg energizing path 34R, and a perceptible level of electrostimulation only by the right leg RL is given. In other words, it can be considered that the motion control unit 48 can give a perceptible level of electrostimulation only on either the left leg LL or the right leg RL by differentiating combinations of the electrodes 32L and 32R or polarity of the electrodes 32L and 32R to which the EMS waveform groups 50L and 50R are applied according to the EMS waveform groups 50L and 50R to be applied.

The specific waveforms of the EMS waveform groups 50L and 50R are not limited to the details of the embodiments. For example, the EMS waveform groups 50L and 50R may consist of only one of the first pulse waveform group 52A and the second pulse waveform group 52B. Further, it is only necessary for the second pulse waveform group 52B to be applied outside the application period Tc (period Th) of the first pulse waveform group 52A in the first cycle Tf, and it is not required to be output every second cycle Tg. For example, a pause period may be inserted between the application period Tc of the first pulse waveform group 52A and the application period Tc of the second pulse waveform group 52B.

The number of block periods Tb provided for the EMS waveform group 50 is not limited. The number of block periods Tb may be singular or may be six or more.

An example in which the target to which electrostimulation is given by the EMS waveform group 50 is a leg has been described thus far. However, specific examples thereof are not particularly limited. For example, the target of electrostimulation may be the abdomen, arms, or other parts of the body in addition to the legs. In any case, it is only necessary for the target of electrostimulation to be a part of the body. In this case, the electrode group 16 is only required to form an energizing path in the target of electrostimulation (a part of the body), and specific examples thereof are not limited to those in the embodiment.

The electrostimulation device 10 is not required to be used in combination with the remote control 14. It means that the electrostimulation device 10 may be operated only by input operations to the device operation units 26A and 26B. In realizing this, the same input operations as those for the remote control operation units 82A-82E of the remote control 14 may be realized by changing the operation time for the device operation units 26A and 26B, the combination of the device operation units 26A and 26B that are subject to input operations, or the like. For example, the same input operation as that for the power supply operation unit 82A of the remote control 14 may be realized by performing a pressing operation on the device operation unit 26A for a predetermined threshold time or longer (hereinafter referred to as "long pressing operation"). In addition to this, for example, the same input operation as that for the menu operation unit 82B of the remote control 14 may be realized by performing a long pressing operation on the device operation unit 26B. Furthermore, for example, the same input operation as that for the switching operation unit 82E of the remote control 14 may be realized by performing a pressing operation on the plurality of device operation units 26A and 26B at the same time. When performing a pressing operation on the device operation units 26A and 26B for less than a predetermined threshold time (short pressing operation), the same input operation as that for the level operation units 82C and 82D of the remote control 14 is realized.

A program including a module corresponding to each block of the device control unit 44 may be stored in a recording medium such as a DVD and installed in the device control unit 44. In addition to this, a program stored in the storage may be read by a processor (CPU or the like) and then executed so as to thereby perform the functions of each block.

The above embodiments and variations are examples. The technical ideas that abstract these should not be interpreted as being limited to the details of the embodiments and variations. The details of the embodiments and variations can be subject to many design changes, such as modification, addition, or deletion of the constituent elements. In the above-described embodiments, the details for which such design changes are possible are emphasized with the notation "embodiment". However, design changes are also allowed for those without such a notation. Hatching applied to a cross section of a drawing does not limit the material of an object to which the hatching is applied. The numerical values referred to in the embodiments and variations (e.g., frequencies corresponding to the first period Tf) naturally include those that can be regarded as the same when errors are considered.

Optional combinations of the above constituting elements are also valid as aspects of the present disclosure. Further, conversion of expressions of the present disclosure among devices, computer programs, recording media storing a computer program, etc., are also valid as aspects of the present disclosure.

### [INDUSTRIAL APPLICABILITY]

The present disclosure relates to an electrostimulation device for giving electrostimulation to a user.

### [REFERENCE SIGNS LIST]

10 electrostimulation device, 16electrode group, 42 signal application unit, 50, 50L, 50R EMS waveform group, 52A first pulse waveform group, 52B
second pulse waveform group, 56 pulse signal

## Claims

1. A muscle electrostimulation program for giving electrostimulation to a muscle, comprising:
a module implemented by a computer of an electrostimulation device provided with a signal application unit that applies a voltage signal to an electrode group, the module being:
a module that repeatedly applies a plurality of pulse waveform groups to the electrode group as the voltage signal by controlling the signal application unit, wherein
the plurality of pulse waveform groups include:
a first pulse waveform group applied intermittently every first cycle corresponding to a frequency of 15 Hz to 25 Hz; and
a second pulse waveform group applied outside an application period of the first pulse waveform group in the first cycle.

2. The muscle electrostimulation program according to claim 1, wherein pulse output time of the first pulse waveform group is longer than pulse output time of the second pulse waveform group.

3. The muscle electrostimulation program according to claim 1, wherein pulse voltage of the first pulse waveform group is higher than pulse voltage of the second pulse waveform group.

4. The muscle electrostimulation program according to claim 1, wherein
pulse output time of the first pulse waveform group is longer than pulse output time of the second pulse waveform group, and
pulse voltage of the first pulse waveform group is higher than pulse voltage of the second pulse waveform group.

5. The muscle electrostimulation program according to any one of claim 1 through claim 4, wherein
the pulse waveform group is repeatedly applied every second cycle, which is shorter than the first cycle, within the same first cycle, and
a part for one of the second cycles in the first cycle serves as an application period for the first pulse waveform group.

6. The muscle electrostimulation program according to any one of claim 1 through claim 5, wherein
the module that repeatedly applies a plurality of pulse waveform groups to the electrode group applies the plurality of pulse waveform groups repeatedly to the electrode group in each of a plurality of consecutive block periods by controlling the signal application unit, and
each of the plurality of block periods is different in at least one of the pulse waveform of the pulse waveform group or the duration of the block period.

7. The muscle electrostimulation program according to any one of claim 1 through claim 6, wherein
the module that repeatedly applies a plurality of pulse waveform groups to the electrode group alternately applies, as the voltage signal, a left leg electric muscle stimulation (EMS) waveform group for giving electrostimulation to a left leg and a right leg EMS waveform group for giving electrostimulation to a right leg to the electrode group by controlling the signal application unit, and
the EMS waveform group is a repetition of the plurality of pulse waveform groups.

8. An electrostimulation device comprising:
an electrode group that gives electrostimulation to a user;
a signal application unit that applies a voltage signal to the electrode group; and
a control unit that executes the muscle electrostimulation program according to any one of claim 1 through claim 7.
